# EUROPÄISCHE PATENTANMELDUNG

(11) **EP 2 801 562 A1**
(43) Veröffentlichungstag der Anmeldung: **12.11.2014**
(21) Anmeldenummer: 13166646.3
(22) Anmeldetag: 06.05.2013
(51) Int. Cl.: C07C 37/50

(54) **Decarboxylierung von 6-Methylsalicylsäure**

(71) Anmelder: LANXESS Deutschland GmbH, 50569 Köln (DE)
(72) Erfinder: Schlummer, Björn, 53225 Bonn (DE); Vogl, Nadine, 50733 Köln (DE); Dreisbach, Claus, 42799 Leichlingen (DE)

(57) **Zusammenfassung**

Die vorliegende Erfindung betrifft ein besonders einfaches Verfahren zur Decarboxylierung von 6-Methylsalicylsäure zu m-Kresol mittels Metall-Katalysatoren oder deren Verbindungen mit Nichtmetallen.

## Beschreibung

Die vorliegende Erfindung betrifft ein besonders einfaches Verfahren zur Decarboxylierung von 6-Methylsalicylsäure (6-MSA).

Aus Berichte der Deutschen Chemischen Gesellschaft, Berlin, Verlag Chemie, Jahrg. 1883, Seite 1963 ist bekannt, 6-Methylsalicylsäure, auch als β-Metahomosalicylsäure bezeichnet, durch Erhitzen mit konzentrierter Salzsäure auf 200°C in Kohlensäure und meta-Kresol zu spalten. Diese Reaktion muss unter erhöhtem Druck durchgeführt werden, um die erforderlichen Reaktionstemperaturen zu realisieren,

Aus WO 2012/178110 A2 ist die Decarboxylierung von 6-MSA durch Erhitzen mit Metallkatalysatoren bekannt. Explizit werden dort 20 g 6-MSA mit 2,5 g Zinkpulver decarboxyliert und 11,35 g m-Kresol in einer Reinheit von über 99,9 % erhalten.

Ausgehend von diesem Stand der Technik bestand die Aufgabe der vorliegenden Erfindung darin, alternative Katalysatoren für die Decarboxylierung von 6-MSA bereit zu stellen. Für industrielle Anwendungen wäre der Aufwand des Einsatzes von 0,0382 Mol Zink für 0,1315 Mol 6-MSA gemäß Beispiel 7 der WO 2012/178110 A2, um daraus 0,1 Mol m-Kresol zu erhalten, nicht rentabel und zudem mit hohem Aufwand hinsichtlich der Entsorgung des verbrauchten Zink-Katalysators verbunden. Für jeden Einsatz müsste neuer Zink-Katalysator bereit gestellt werden und dieser nach dem Einsatz entsorgt werden, wodurch allenfalls eine Batch-Fahrweise möglich wäre.

Es wurde nun überraschend gefunden, dass Metalle der Reihe Fe, Cu, Pd, Ni, Mo, Ru und Co oder deren Verbindungen mit Nichtmetallen der Reihe Oxide, Chloride, Acetate oder deren Komplexe mit CO oder Dibenzylidenaceton in deutlich effektiverer Weise 6-MSA decarboxylieren.

Gegenstand der vorliegenden Erfindung ist ein Verfahren zur Decarboxylierung von 6-MSA, dadurch gekennzeichnet, dass man wenigstens einen Katalysator der Reihe Fe, Cu, Pd, Ni, Mo, Ru oder Co oder deren Verbindungen mit Nichtmetallen der Reihe Oxide, Chloride, Acetate oder deren Komplexe mit CO oder Dibenzylidenaceton einsetzt.

Zur Klarstellung sei angemerkt, dass vom Rahmen dieser Erfindung alle nachfolgend aufgeführten, allgemeinen oder in Vorzugsbereichen genannten Definitionen und Parameter in beliebigen Kombinationen umfasst sind.

Bevorzugtes Decarboxylierungsprodukt ist meta-Kresol (m-Kresol).

In einer Ausführung betrifft die vorliegende Erfindung ein Verfahren zur Herstellung von m-Kresol, indem man ausgehend von 6-MSA wenigstens einen Katalysator der Reihe Fe, Cu, Pd, Ni, Mo, Ru oder Co oder deren Verbindungen mit Nichtmetallen der Reihe Oxide, Chloride, Acetate oder deren Komplexe mit CO oder Dibenzylidenaceton einsetzt.

Gegenstand der vorliegenden Anmeldung ist auch die Verwendung wenigstens eines Katalysators der Reihe Fe, Cu, Pd, Ni, Mo, Ru oder Co oder deren Verbindungen mit Nichtmetallen der Reihe Oxide, Chloride oder Acetate oder deren Komplexe mit CO oder Dibenzylidenaceton zur Decarboxylierung von 6-MSA.

In einer Ausführungsform betrifft die vorliegende Erfindung ein Verfahren zur Decarboxylierung von 6-MSA, dadurch gekennzeichnet, dass man wenigstens einen Katalysator der Reihe Fe, Cu, Pd, Ru oder Co oder deren Verbindungen mit Nichtmetallen der Oxide oder Chloride einsetzt.

In einer Ausführungsform betrifft die vorliegende Erfindung ein Verfahren zur Decarboxylierung von 6-MSA, dadurch gekennzeichnet, dass man wenigstens einen Katalysator der Reihe Fe, Cu, Pd auf Aktivkohle, Ru auf Aluminiumoxid, Raney-Co, Cu₂O oder FeCl₃ einsetzt.

In einer Ausführung der vorliegenden Erfindung wird Cu₂O eingesetzt.

In einer Ausführung der vorliegenden Erfindung wird FeCl₂ eingesetzt.

In einer Ausführung der vorliegenden Erfindung wird FeCl₃ eingesetzt.

In einer Ausführung der vorliegenden Erfindung wird Cu(OAc)₂ eingesetzt.

In einer Ausführung der vorliegenden Erfindung wird Pd(OAc)₂ eingesetzt.

In einer Ausführung der vorliegenden Erfindung wird Pd₂dba₃ eingesetzt.

In einer Ausführung der vorliegenden Erfindung wird NiCl₂ eingesetzt.

In einer Ausführung der vorliegenden Erfindung wird Fe eingesetzt.

In einer Ausführung der vorliegenden Erfindung wird Cu eingesetzt.

In einer Ausführung der vorliegenden Erfindung wird Pd/C 5 % eingesetzt.

In einer Ausführung der vorliegenden Erfindung wird Pd/C10 % eingesetzt.

In einer Ausführung der vorliegenden Erfindung wird Raney-Ni eingesetzt.

In einer Ausführung der vorliegenden Erfindung wird Mo(CO)₆ Molybdänhexacarbonyl eingesetzt.

In einer Ausführung der vorliegenden Erfindung wird Ru/Aluminiumoxid eingesetzt.

In einer Ausführung der vorliegenden Erfindung wird Raney-Co eingesetzt.

Erfindungsgemäß steht Ac für Acetat, Ph steht für Phenyl und dba₃ steht für Dibenzylidenaceton, so dass der Katalysator Pd₂dba₃ die folgende chemische Strukturformel aufweist:

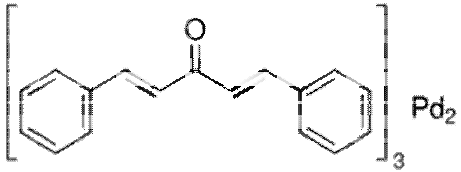

In einer Ausführungsform kommen geträgerte Katalysatoren zum Einsatz. Geträgerte Katalysatoren bestehen aus wenigstens einer der oben genannten katalytisch aktiven Komponenten, die auf einem Trägermaterial dispergiert ist. Dieses Trägermaterial zeichnet sich meist durch eine große spezifische Oberfläche aus, so dass sich die aktive Komponente fein verteilen kann. Das Trägermaterial kann katalytisch inert sein, ist jedoch oft an der Bildung einer katalytisch aktiven Oberfläche beteiligt. Die katalytisch aktive Komponente kann im Falle geträgerter Katalysatoren in drei verschiedenen Formen vorliegen:
- Der Träger agiert nur als Dispersionsmittel, die katalytisch aktive Komponente bleibt chemisch unverändert.
- Es bildet sich eine Mischphase, in der Träger und aktive Komponente teilweise eine chemische Verbindung eingehen.
- Es entsteht ein fester Verbund, in dem sich die aktive Komponente im Träger löst.

Ein bevorzugter geträgerter Katalysator ist Ru/Aluminiumoxid, worin das Aluminiumoxid als Träger fungiert.

In einer Ausführungsform wird das erfindungsgemäße Verfahren lösungsmittelfrei durchgeführt und ist damit dem Verfahren des Standes der Technik in der Durchführung technisch überlegen.

In einer Ausführungsform wird das erfindungsgemäße Verfahren bei einer Temperatur im Bereich von 180°C-200°C, bevorzugt im Bereich von 190°C-200°C durchgeführt. Die experimentellen Daten zur vorliegenden Erfindung wurden 195°C durchgeführt.

In einer Ausführungsform kann 6-MSA auf petrochemischer Basis oder aus mikrobiologischen Syntheseprozessen eingesetzt werden.

In einer Ausführungsform kann das erfindungsgemäße Verfahren in Gegenwart der 0,5 bis 15-fachen Gewichtsmenge Wasser, bezogen auf die einzusetzende Menge 6-MSA durchgeführt werden. Bevorzugt beträgt diese Menge das 0,8- bis 6-fache.

In einer Ausführungsform kann das erfindungsgemäße Verfahren in Gegenwart der bis zu 15-fachen Gewichtsmenge einer oder mehrerer weiterer Säuren bezogen auf die eingesetzte Menge 6-MSA durchgeführt werden. Vorzugsweise beträgt diese Menge das 0,01- bis 5-fache.

Die 6-MSA erhitzt man gegebenenfalls in Gegenwart von Wasser und/oder weiterer Säuren, vorzugsweise auf eine Temperatur von 190 bis 200°C. Wenn die Reaktionstemperatur bei Normaldruck über der Siedetemperatur des Reaktionsgemisches liegt, ist es erforderlich, die Decarboxylierung in einem druckfesten Reaktor, bevorzugt einen Autoklaven, durchzuführen.

Die Reaktionszeiten können in einem weiten Bereich variiert werden und vorzugsweise im Bereich von 1 bis 60 Stunden liegen. Im Allgemeinen kann bei höheren Reaktionstemperaturen mit kürzeren Reaktionszeiten gearbeitet werden, als bei tieferen Reaktionszeiten. Tiefere Temperaturen, vorzugsweise solche von 80 bis 150°C, sind dann vorteilhaft, wenn große Wassermengen und kleinere Mengen an oder keine weiteren Säuren eingesetzt werden.

Es sind wesentliche Merkmale des erfindungsgemäßen Verfahrens, dass man es ohne Zusätze von Wasser, Säuren, Basen und organischen Lösungsmitteln durchführt.

Das nach der Durchführung der erfindungsgemäßen Decarboxylierung vorliegende Reaktionsmedium kann direkt destilliert werden, um das bevorzugte Reaktionsprodukt m-Kresol zu isolieren. Man erhält so im Allgemeinen über 97 % reines m-Kresol. Der Siedepunkt von m-Kresol liegt bei 203°C, so dass entsprechend hohe Temperaturen für die Destillation des Reaktionsmediums gewählt werden müssen.

Im Hinblick auf den eingangs geschilderten Stand der Technik ist es beim erfindungsgemäßen Verfahren ausgesprochen überraschend, dass es ohne Zusätze von Basen und ohne organische Lösungsmittel gelingt, mit guten Ausbeuten aus 6-MSA selektiv die Carboxygruppe abzuspalten und bevorzugt das Decarboxylierungsprodukt m-Kresol in hohen Ausbeuten und hohen Reinheiten zu erhalten.

Das bevorzugte Reaktionsprodukt m-Kresol ist ein wertvolles Zwischenprodukt zur Herstellung von Pflanzenschutz- und Pharmawirkstoffen. m-Kresol findet als Fungizid in der Landwirtschaft Anwendung. Kresole werden auch verwendet, um daraus Kunst- und Farbstoffe, Kunstharze (Kresolharze) und Arzneimittel herzustellen.

### Beispiele

### Allgemeine Arbeitsvorschrift:

1,0 g (6,6 mmol) 6-Methylsalicylsäure wurden mit der angegebenen Menge des zu verwendenden Katalysators innig vermengt und dann ohne Zugabe von Lösungsmittel unter inerter Atmosphäre auf 195 °C erhitzt. Das Gemisch wurde 6 h bei dieser Temperatur gehalten und dann auf Raumtemperatur abgekühlt. Das erhaltene Produktgemisch wurde mit 50 g Toluol extrahiert und das erhaltene Produktgemisch nach Silylierung mittels Gaschromatographie untersucht und die Produktverhältnisse bestimmt.

Die Ergebnisse der einzelnen Versuche sind in Tabelle 1 dargestellt. In Tabelle 1 bedeutet 5g Versuch, dass in obiger Versuchsvorschrift 5g 6-Methylsalicylsäure mit 100 mol-% Katalysator umgesetzt wurden. Einzig im Falle des Einsatzes von Pd/C 5% wurden für die Umsetzung von 5g 6-MSA nur 18 mol-% des Katalysators eingesetzt (*).

Die Katalysatoren wurden in Form der aufgeführten Metallverbindungen oder als Metalle, gegebenenfalls als geträgerte Katalysatoren, oder in Form von Metallkomplexen (Pd₂dba₃ bzw. Mo(CO)₆) eingesetzt.

Für die Ermittlung der Ausbeute wurde die Vorschrift im fünffachen Maßstab durchgeführt und die erhaltene organische Produktphase vollständig vom Lösungsmittel befreit, um die Produktmenge per Auswaage zu bestimmen.

Das Produkt wurde dann nach Silylierung mittels Gaschromatographie untersucht.

Die Ergebnisse des mit dem jeweiligen Katalysator erzielten Umsatzes ist in Tabelle 1 dargestellt. Jeweils angegeben ist der Umsatz von 6-MSA zu m-Kresol in Prozent.

**Tabelle 1:**

| **Eingesetzte Menge des Katalysators** | | | | |
|---|---|---|---|---|
| **Katalysator** | 100 mol-% | 50 mol-% | 10 mol-% | 5g Versuch |
| HCl (37%) | 41 | | | |
| Kein Katalysator | 34 | | | 12 |

| **Metallverbindungen/Komplexe** | | | | |
|---|---|---|---|---|
| Cu₂O | 100 | 88 | 86 | 35 |
| FeCl₂ | 77 | 79 | 100 | 100 |
| FeCl₃ | 100 | 42 | | |
| Cu(OAc)₂ | 52 | | | |
| Pd₂dba₃ | 71 | 86 | | |
| NiCl₂ | 87 | 76 | 100 | |

| **Metalle/Komplexe** | | | | |
|---|---|---|---|---|
| | | | | |
| Fe | 100 | 100 | 100 | 84 |
| Cu | 85 | 100 | | |
| Pd/C 5% | | 100 | | 25* |
| Pd/C 10% | | 100 | | |
| Raney-Ni | 49 | 58 | | |
| Mo(CO)₆ | 86 | 67 | | |
| Ru/Alox | 100 | 100 | | |
| Raney-Co | 100 | 100 | 80 | |

100 mol-% bedeuten, dass relativ zu der eingesetzten Molmenge 6-Methylsalicylsäure die gleiche Molmenge (100 mol-%) des Katalysators eingesetzt wurde.50 mol-% bedeuten, dass relativ zu der eingesetzten Molmenge 6-Methylsalicylsäure die halbe molare Menge (50 mol-%) des Katalysators eingesetzt wurde. 10 mol-% bedeuten, dass relativ zu der eingesetzten Molmenge 6-Methylsalicylsäure ein Zehntel der molaren Menge (10 mol-%) des Katalysators eingesetzt wurde.

Raney^{®}-Nickel, CAS Nummer 7440-02-0, zu beziehen bei W.R.Grace & Co.

Raney^{®}-Kobalt, CAS Nummer 7440-48-4, zu beziehen bei W.R.Grace & Co.

Ru/Alox ist ein Ruthenium Aluminiumoxid Katalysator mit der CAS Nummer 7440-18-8 zu beziehen bei Heraeus Precious Metals GmbH & Co. KG, Hanau, Deutschland.

Pd/C 10% ist ein Palladium auf Aktivkohle 10% Katalysator mit der CAS Nummer 7440-05-3 zu beziehen bei Heraeus Precious Metals GmbH & Co. KG, Hanau, Deutschland.

Pd/C 5% ist ein Palladium auf Aktivkohle 5% Katalysator mit der CAS Nummer: 7440-05-3 zu beziehen bei Heraeus Precious Metals GmbH & Co., Hanau, Deutschland KG.

Kupferacetat Cu(OAc)₂ ; CAS Nummer 142-71-2 (wasserfrei) zu beziehen bei Sigma-Aldrich Chemistry, Deutschland

Mo(CO)₆ ist ein Molybdänhexacarbonyl Katalysator mit der CAS Nummer 13939-06-5 zu beziehen bei Sigma Aldrich Chemistry, Deutschland.

Pd₂dba₃ ist ein Tris(dibenzylideneacetone)dipalladium(O)-Katalysator mit der CAS Nummer 51364-51-3, zu beziehen bei Alfa Aesar GmbH & Co. KG, Karlsruhe, Deutschland.

Die mit den erfindungsgemäßen Katalysatoren erzielten Ergebnisse zeigen überraschenderweise eine gegenüber dem Stand der Technik ausgesprochen hohe Aktivität auch bei niedrigen Beladungen von 10 mol-%. Die geträgerten Katalysatoren ermöglichen eine Durchführung in kontinuierlichen Prozessvarianten. Die eingesetzten Metalle wie Eisen und Kupfer zeigen im Vergleich zu Zink deutlich verminderte pyrophore Eigenschaften. Die eingesetzten Katalysatoren zeigen einen deutlich niedrigeren Aufwand zur Entsorgung im Vergleich zu Zink, bzw. lassen sich in den geträgerten Varianten wiederverwenden.

## Patentansprüche

1. Verfahren zur Decarboxylierung von 6-MSA, **dadurch gekennzeichnet, dass** man wenigstens einen Katalysator der Reihe Fe, Cu, Pd, Ni, Mo, Ru oder Co oder deren Verbindungen mit Nichtmetallen der Reihe Oxide, Chloride, Acetate oder deren Komplexe mit CO oder Dibenzylidenaceton einsetzt.

2. Verfahren gemäß Anspruch 1, **dadurch gekennzeichnet, dass** man wenigstens einen Katalysator der Reihe Fe, Cu, Pd, Ru oder Co oder deren Verbindungen mit Nichtmetallen der Oxide oder Chloride einsetzt.

3. Verfahren gemäß der Ansprüche 1 oder 2, **dadurch gekennzeichnet, dass** man wenigstens einen Katalysator der Reihe Fe, Cu, Pd auf Aktivkohle, Ru auf Aluminiumoxid oder Ranay-Co oder Cu₂O oder FeCl₃ einsetzt.

4. Verfahren gemäß Anspruch 1, **dadurch gekennzeichnet, dass** man Cu₂O einsetzt.

5. Verfahren gemäß Anspruch 1, **dadurch gekennzeichnet, dass** man FeCl₂ einsetzt.

6. Verfahren gemäß Anspruch 1, **dadurch gekennzeichnet, dass** man FeCl₃ einsetzt.

7. Verfahren gemäß Anspruch 1, **dadurch gekennzeichnet, dass** man Cu(OAc)₂ einsetzt.

8. Verfahren gemäß Anspruch 1, **dadurch gekennzeichnet, dass** man Pd(OAc)₂ einsetzt.

9. Verfahren gemäß Anspruch 1, **dadurch gekennzeichnet, dass** man Pd₂dba₃ einsetzt worin dba₃ für Dibenzylidenaceton steht.

10. Verfahren gemäß Anspruch 1, **dadurch gekennzeichnet, dass** man NiCl₂ einsetzt.

11. Verfahren gemäß Anspruch 1, **dadurch gekennzeichnet, dass** man Fe einsetzt.

12. Verfahren gemäß Anspruch 1, **dadurch gekennzeichnet, dass** man Cu einsetzt.

13. Verfahren gemäß Anspruch 1, **dadurch gekennzeichnet, dass** man Pd/C 5 % oder Pd/C10 % einsetzt.

14. Verfahren gemäß Anspruch 1, **dadurch gekennzeichnet, dass** man Raney-Ni oder Raney-Co einsetzt.

15. Verfahren gemäß Anspruch 1, **dadurch gekennzeichnet, dass** man Ru/Aluminiumoxid einsetzt.

16. Verfahren gemäß Anspruch 1, **dadurch gekennzeichnet, dass** man Mo(CO)₆ einsetzt.

17. Verfahren gemäß der Ansprüche 1 bis 16, **dadurch gekennzeichnet, dass** geträgerte Katalysatoren eingesetzt werden.

18. Verfahren gemäß der Ansprüche 1 bis 17, **dadurch gekennzeichnet, dass** das Reaktionsprodukt m-Kresol ist.

19. Verfahren gemäß Anspruch 18, **dadurch gekennzeichnet, dass** man das m-Kresol durch Destillation des nach der Decarboxylierung erhaltenen Reaktionsmediums erhält.
